# EUROPEAN PATENT SPECIFICATION

(11) **EP 1 417 970 B1**
(45) Date of publication and mention of the grant of the patent: **19.11.2008**
(21) Application number: 03007761.4
(22) Date of filing: 04.04.2003
(51) Int. Cl.: A61K 31/728, A61K 31/711, A61K 36/28, A61K 36/53, A61K 47/00

(54) **Anti-allergic, anti-bacteric and anti-redden compound for eyes**
Antiallergische, antibakterielle und rötungsmindernde Augenzusammensetzung
Composition antiallergique et antibacterienne pour les yeux réduisant les rougeurs des yeux

(30) Priority: 06.11.2002 IT MI20022353
(43) Date of publication of application: 12.05.2004
(73) Proprietor: Farmaceutici S.R.L., 15057 Tortona, Alessandria (IT)
(72) Inventor: Bonabello, Elvio, 15067 Novi Ligure (IT)
(74) Representative: Cicogna, Franco

(56) References cited:
- EP-A- 0 698 388
- WO-A-01/49304
- WO-A-99/58095
- PATENT ABSTRACTS OF JAPAN vol. 2003, no. 03, 5 May 2003 (2003-05-05) & JP 2002 326941 A (MORITA NAOMASA;HIROSE KIYOSHI; TOCHITANI FUJIO; TANETANI MASAKO; TEIKA), 15 November 2002 (2002-11-15)
- DATABASE WPI Section Ch, Week 198847 Derwent Publications Ltd., London, GB; Class B04, AN 1988-332397 XP002280185 & HU 46 229 A (SEMMELWEIS ORVOSTUD) 28 October 1988 (1988-10-28)
- DATABASE MEDLINE [Online] US NATIONAL LIBRARY OF MEDICINE (NLM), BETHESDA, MD, US; 15 December 1999 (1999-12-15), GARBACKI N ET AL: "Anti-inflammatory and immunological effects of Centaurea cyanus flower-heads." XP002270550 Database accession no. NLM10624883 & JOURNAL OF ETHNOPHARMACOLOGY. IRELAND 15 DEC 1999, vol. 68, no. 1-3, 15 December 1999 (1999-12-15), pages 235-241, ISSN: 0378-8741
- PAPA V ET AL: "COMPARISON OF HYPOTONIC AND ISOTONIC SOLUTIONS CONTAINING SODIUM HYALURONATE ON THE SYMPTOMATIC TREATMENT OF DRY EYE PATIENTS" OPHTHALMOLOGICA, KARGER, BASEL, CH, vol. 215, no. 2, March 2001 (2001-03), pages 124-127, XP001105457 ISSN: 0030-3755
- DILIKA, F. ET AL: "Antibacterial activity of linoleic and oleic acids isolated from Helichrysum pedunculatum: a plant used during circumcision rites" FITOTERAPIA (2000), 71(4), 450-452, 2000, XP002270549

## Description

### BACKGROUND OF THE INVENTION

The present invention relates to a compound composition for eyes, having an anti-allergic, anti-bacteric and anti-redden activity.

As is known, a problem which is frequently encountered in making compounds for the eyes, such as eyewash solutions and the like, is that the made compound cannot be properly tolerated by eyes.

The document WO 99/58095 discloses a pharmaceutical composition, for oral administering, including sea water and hydroxyethyl cellulose.

WO 01/49304 claims the use for treating and cleaning the eye and its appendages of aqueous ionic solutions obtained from sea water.

### SUMMARY OF THE INVENTION

Accordingly, the aim of the present invention is to solve the above mentioned problem.

Within the scope of the above mentioned aim, a main object of the invention is to provide such a compound/eyewash solution for the eyes, which has an anti-allergic, anti-bacteric and anti-redden activity, without having any side effects as found in prior eye compounds.

Yet another object of the present invention is to provide such an eye compound comprising an eyewash solution having high efficiency and tolerability, and which can also be used for comparatively long periods of times.

Yet another object of the present invention is to provide such an eye compound which has chemical characteristics perfectly analogous to those of a human tear.

Yet another object of the present invention is to provide such an eye compound which has a mucin-like viscosity, thereby providing mechanic and lubricating characteristics analogous to those found in the human tear.

According to one aspect of the present invention, the above mentioned aim and objects, as well as yet other objects, which will become more apparent hereinafter, are achieved by an eye compound having an anti-allergic, anti-bacteric and anti-redden activity, according to claim 1.

Further characteristics and advantages of the present invention will become more apparent hereinafter from the following detailed disclosure of an eye compound having an anti-allergic activity, which is made with reference to a preferred embodiment thereof, given only by a mere indicative example.

In this connection, it should be apparent that the eye compound according to the invention is susceptible to many modifications and variations, all of which will come within the scope of the invention.

The eye compound according to the invention, having an anti-allergic, anti-bacteric and anti-redden activity, has the main feature that it is made of a filtered sea water base, which has been purified and made isotonic by adding distilled water thereto.

To said base are further added active substances having specific functions.

In particular, is added an active substance having mucin-like characteristics, namely hydroxypropyl-methyl cellulose, allowing the compound to strongly adhere to the cornea, thereby providing a thin protective film.

The latter allows to greatly reduce the water evaporation rate, while assuring a longer holding time for the active substances.

The above mentioned active substance is added in a rate from 0.01 to 5%.

The compound is further added with yet another active substance operating for embedding water molecules therein, thereby increasing its viscoelasticity in order to provide a better sliding of the eyelid, while providing a tear replacement function.

This substance, which is included in a rate from 0.01 to 5%, comprises sodium hyaluronate, a natural water soluble bipolymer.

In order to reduce the redden of conjunctiva centaura cyanus L. (cornflower), having astringent and anti-edematic properties is further added in a rate from 0.001 and 3%.

Moreover, for providing an anti-bacteric and healing activity, is further added helychrysum ictalicum (helichrysum) in a rate from 0.001 to 3%.

To provide a great anti-inflammatory and decongestant activity of the conjunctiva is moreover added sea cineraria L. (senecio cineraria) in a rate from 0.001 to 3%.

For providing further anti-inflammatory and anti-bacteric properties, allowing the eye compound to be used for eye washes, in the case of blepharitis, it is further added euphrasia officinalis L. (euphrasia) in a rate from 0.01 to 3%.

For providing anti-inflammatory, anti-edema and anti-allergic properties, it is further added calendula officinalis L. (calendula) in a rate from 0.001 to 3%.

Anyhow, the greatest amount of the eye compound according to the invention is constituted by sea water, which is made sterile and brought to an isotonic level with respect to the body fluids.

As is known, the human tear has chemical-physical characteristics which can be overlapped on those of the human plasma.

Since biologic life has derived from the sea, it would be apparent that sea water, as suitably diluted and brought to an isotonic level, would be susceptible to also become like plasma.

Since it is not possible to make a solution identical to a tear in a laboratory in order to improve a product suitable to carry all the active substances, but which also has a tear substitution function, it has been found that sterile and isotonic sea water allows to solve this problem.

From the above disclosure it should be apparent that the invention fully achieves the intended aim and objects.

In particular, the fact is to be pointed out that an eye compound has been provided which is based on the use of isotonic sea water allowing to provide better tolerability and efficiency of the therapeutical powers of the included active substances.

The recent scientific results, moreover, have demonstrated the uniqueness in carrying aquaporins of the ocular bulb.

The invention, as disclosed, is susceptible to several modifications and variations, all of which will come within the scope of the invention.

Moreover, all the component substances could be replaced by other technically equivalent substances.

## Claims

1. An eye compound, having an anti-allergic, anti-bacteric and anti-redden activity, said eye compound comprising a base constituted by isotonic sea water in which active substances are present, **characterized in that** said eye compound further comprises hydroxypropyl-methyl-cellulose in a rate from 0.01 to 5%, thereby allowing said eye compound to strongly adhere to the eye cornea and provide a thin protective film thereon reducing the water evaporation rate, while assuring a long holding time, said compound further comprising at least an active substance operating for embedding water molecules therein, thereby increasing the viscoelasticity of said compound to provide a better sliding of the eyelid while providing a tear replacement function and comprising sodium hyaluronate being added in a total rate from 0.01 to 5%.

2. An eye compound according to claim 1, **characterized in that** said compound further comprises at least one or more of euphrasia in a rate from 0.001 to 3%, centaurea cyanus L, in a rate from 0.001 to 3%, helichrysum italicum, in a rate from 0.001 to 3%, cineraria maritima L, in a rate from 0.001 to 3%, and calendula officinalis L, in a rate from 0.001 to 3%.

## Patentansprüche

1. Augenzusammensetzung, die eine antiallergische, antibakterielle und rötungsmindernde Aktivität aufweist, wobei die Augenzusammensetzung einen Grundstoff enthält, gebildet aus isotonischem Meerwasser, in dem aktive Substanzen vorhanden sind, **dadurch gekennzeichnet, dass** die Augenzusammensetzung ferner Hydroxypropylmethylzellulose in einem Anteil von 0,01 bis 5 % enthält, wodurch es der Augenzusammensetzung ermöglicht wird, ausgezeichnet an der Hornhaut des Auges zu haften und einen dünnen, schützenden Film darauf bereitzustellen, wodurch die Geschwindigkeit der Wasserverdunstung herabgesetzt ist, während gleichzeitig eine lange Verweilzeit gewährleistet ist, wobei die Zusammensetzung ferner mindestens eine aktive Substanz umfasst, die darin den Einschluss von Wassermolekülen bewirkt, wodurch die Viskoelastizität von der Zusammensetzung erhöht ist, um ein besseres Gleiten von dem Augenlid bereitzustellen, während zur gleichen Zeit eine Tränenersatzfunktion bereitgestellt ist, und die Natriumhyaluronat umfasst, das in einem Gesamtanteil von 0,01 bis 5 % hinzugefügt ist.

2. Augenzusammensetzung nach Anspruch 1, **dadurch gekennzeichnet, dass** die Zusammensetzung ferner mindestens ein oder mehrere von Euphrasia, in einem Anteil von 0,001 bis 3 %, Centaurea cyanus L, in einem Anteil von 0,001 bis 3 %, Helichrysum italicum, in einem Anteil von 0,001 bis 3 %, Vineraria maritima L, in einem Anteil von 0,001 bis 3 %, und Calendula officinalis L, in einem Anteil von 0,001 bis 3 %, umfasst.

## Revendications

1. Composé ophtalmique ayant une activité anti-allergique, anti-bactérienne et anti-rubéfaction, ledit composé ophtalmique comprenant une base constituée d'eau de mer isotonique dans laquelle des substances actives sont présentes, **caractérisé en ce que** ledit composé ophtalmique comprend en outre de l'hydroxypropyl-méthyl-cellulose en un pourcentage compris entre 0,01 et 5 %, ce qui permet au dit composé ophtalmique d'adhérer fortement sur la cornée de l'oeil et de fournir un film protecteur mince sur celle-ci en réduisant le taux d'évaporation de l'eau, tout en assurant une longue tenue, ledit composé comprenant en outre au moins une substance active dont la fonction est l'incrustation de molécules d'eau dans celle-ci, ce qui augmente la viscoélasticité dudit composé afin de fournir un meilleur glissement de la paupière tout en ayant une fonction de remplacement des larmes et comprenant de l'hyaluronate de sodium qui est ajouté en un pourcentage total compris entre 0,01 et 5 %.

2. Composé ophtalmique selon la revendication 1, **caractérisé en ce que** ledit composé comprend en outre au moins l'un, ou plusieurs, parmi l'euphraise (euphrasia) en un pourcentage compris entre 0,001 et 3 %, la centaurée bleue L (centaurea cyanus L), en un pourcentage compris entre 0,001 et 3 %, l'immortelle d'Italie (helichrysum italicum) en un pourcentage compris entre 0,001 et 3 %, la cinéraire maritime (cineraria maritima L) en un pourcentage compris entre 0,001 et 3 % et le souci (calendula officinalis L) en un pourcentage compris entre 0,001 et 3 %.
